# FASCICULE DE BREVET EUROPEEN

(11) **EP 2 760 811 B1**
(45) Date de publication et mention de la délivrance du brevet: **06.01.2016**
(21) Numéro de dépôt: 12773066.1
(22) Date de dépôt: 20.09.2012
(51) Int. Cl.: C07C 17/20, C07C 21/18

(54) **PROCEDE DE FABRICATION DU 2,3,3,3-TETRAFLUOROPROPENE**
VERFAHREN ZUR HERSTELLUNG VON 2,3,3,3-TETRAFLUORPROPEN
METHOD FOR MANUFACTURING 2,3,3,3-TETRAFLUOROPROPENE

(30) Priorité: 27.09.2011 FR 1158604
(43) Date de publication de la demande: 06.08.2014
(73) Titulaire: Arkema France, 92700 Colombes (FR)
(72) Inventeur: PIGAMO, Anne, F-69340 Francheville (FR); DOUCET, Nicolas, F-69007 Lyon (FR); WENDLINGER, Laurent, F-69510 Soucieu En Jarrest (FR)
(74) Mandataire: Dang, Doris
(86) Numéro de dépôt international: PCT/FR2012/052097
(87) Numéro de publication internationale: WO 2013/045791

(56) Documents cités:
- WO-A1-2009/003084
- WO-A1-2011/077191
- WO-A2-2010/123154

## Description

La présente invention concerne un procédé de fabrication du 2,3,3,3-tetrafluoropropène comprenant au moins une étape de fluoration en phase gaz en présence d'un catalyseur.

Le 2,3,3,3-tetrafluoropropène (HFO-1234yf), du fait de son faible pouvoir de réchauffement Global Warming Potential, est considéré comme un candidat potentiel au remplacement du HFC-134a dans la climatisation automobile.

Le 2,3,3,3-tetrafluoropropène peut être obtenu à partir du 1,2,3,3,3-pentafluoropropène (HFO-1225ye) en faisant réagir le HFO-1225ye avec de l'hydrogène en présence d'un catalyseur d'hydrogénation pour donner du 1,1,1,2,3-pentafluoropropane (HFC-245eb) ; le HFC-245eb ainsi formé est ensuite soumis à une étape de déhydrofluoration en présence de l'hydroxyde de potassium (Knunyants et al., Journal of Academy of Sciences of the USSR, page 1312-1317, August, 1960).

Le 2,3,3,3-tetrafluoropropène peut également être obtenu en faisant réagir le 2-chloro,3,3,3-trifluoropropène (HCFO-1233xf) avec de l'HF en présence d'un catalyseur pour donner dans un premier temps le 1,1,1,2-tetrafluoro,2-chloropropane (HCFC-244bb), puis le HCFC-244bb réagit avec de l'HF sur un deuxième catalyseur (WO 2007/079431).

Le 2,3,3,3-tetrafluoropropène peut également être obtenu à partir des pentachloropropanes ou tetrachloropropènes en passant par le 2-chloro,3,3,3-trifluoropropène comme intermédiaire.

Le document WO 2010/123154 décrit un procédé de fabrication du HFO-1234yf en faisant réagir le HCFO-1233xf avec de l'HF en présence d'oxygène à l'aide d'un catalyseur d'oxyde de chrome de formule CrOₘ, avec 1,5 < m < 3, éventuellement fluoré. Ce document enseigne que pour obtenir une bonne sélectivité en HFO-1234yf, la température de réaction doit être comprise entre 330 et 380°C à une pression de 0,08 à 0,2 MPa avec un ratio molaire d'oxygène par rapport au HCFO-1233xf compris entre 0,1 et 1 et un ratio molaire d'HF par rapport au HCFO-1233xf compris entre 4 et 30.

Le document WO 2010/123154 ne s'intéresse qu'à la sélectivité du HFO-1234yf pour une durée de réaction très faible (maximum 45 heures). Ainsi, la conversion n'est que de 6,2 % à l'exemple 3 après 45 heures de réaction.

Or, pour qu'un procédé soit viable industriellement non seulement la sélectivité doit être élevée mais aussi la conversion. En outre, les performances doivent être quasi-constantes dans le temps.

La demanderesse a maintenant mis au point un procédé de fabrication du 2,3,3,3-tetrafluoropropène pouvant être mis en oeuvre industriellement et ne présentant pas les inconvénients de l'art antérieur. Plus précisément, la présente invention fournit un procédé de fabrication du 2,3,3,3-tetrafluoropropène à partir des halopropanes de formules CX₃CHClCH₂X et CX₃CFXCH₃, halopropènes de formules CX₃CCl=CH₂, CClX₂CCNCH₂ et CX₂=CClCH₂X, avec X représentant indépendamment un atome de fluor ou de chlore.

Le procédé selon la présente invention comprend au moins une étape (FCO) au cours de laquelle du 2-chloro-3,3,3-trifluoro-1-propène éventuellement en mélange avec au moins un halopropane(s) de formules CX₃CHClCH₂X et CX₃CFXCH₃ et/ou au moins un halopropène(s) de formules CClX₂CCNCH₂ et CX₂=CClCH₂X, avec X représentant indépendamment un atome de fluor ou de chlore, réagit ou réagissent avec de l'HF en phase gazeuse en présence d'un catalyseur de fluoration, à une température comprise entre 320 et 420°C, et d'un ratio molaire d'oxygène par rapport au 2-chloro-3,3,3-trifluoro-1-propène, supérieur à 1 et inférieur ou égal à 2,5 et un ratio molaire HF par rapport à la totalité des composés organiques à réagir compris entre 5 et 40.

On entend par composés organiques des composés comprenant au moins du carbone, du chlore, éventuellement au moins un des éléments choisis parmi l'hydrogène et le fluor.

Les composés organiques incluent les produits de départ et les intermédiaires.

Selon un mode de réalisation, le produit de départ est choisi parmi le 2,3-dichloro-1,1,1-trifluoropropane (HCFC-243db), le 1,1,1,2,3-pentachloropropane (HCC-240db), le 2-chloro-3,3,3-trifluoro-1-propène (HCFO-1233xf) et/ou le 1,1,2,3-tetrachloropropène (HCO-1230xa).

Selon un mode de réalisation, le 2-chloro-3,3,3-trifluoro-1-propène représente plus de 20 % en poids du ou des composés organiques à réagir avec de l'HF dans l'étape (FCO) de la présente invention.

Selon un mode de réalisation préféré de l'invention, le HFO-1234yf est obtenu à partir du pentachloropropane, avantageusement le 1,1,1,2,3-pentachloropropane et le procédé comprend une étape (FCO) au cours de laquelle le pentachloropropane et/ou du 2-chloro-3,3,3-trifluoro-1-propène réagit ou réagissent avec de l'HF en phase gazeuse en présence de l'oxygène et d'un catalyseur de fluoration.

Quel que soit le mode de réalisation le ratio molaire d'oxygène par rapport au 2-chloro-3,3,3-trifluoro-1-propène à réagir dans l'étape (FCO) est de préférence compris entre 1,25 et 2,5. Le ratio molaire HF par rapport à la totalité des composés organiques à réagir dans l'étape (FCO) est de préférence compris entre 10 et 40. La température de la réaction de fluoration de l'étape (FCO) est de préférence comprise entre 340 et 400°C.

L'étape (FCO) est en général mise en oeuvre à une pression comprise entre 0,5 et 20 bar, de préférence entre 1 et 7 bar.

Le catalyseur utilisé peut être massique ou supporté. Le catalyseur peut être à base d'un métal notamment d'un métal de transition ou un dérivé oxyde, halogénure ou oxyhalogénure d'un tel métal. Des catalyseurs sont par exemple FeCl₃, oxyfluorure de chrome, NiCl₂, CrF₃ et leurs mélanges.

D'autres catalyseurs possibles sont des catalyseurs supportés sur carbone ou à base de magnésium tels que les dérivés de magnésium notamment des halogénures tel que MgF₂ ou des oxyhalogénures de magnésium tel que les oxyfluorures ou à base d'aluminium comme l'alumine, l'alumine activée ou les dérivés d'aluminium notamment des halogénures, tel que AlF₃ ou oxyhalogénures d'aluminium tel que oxyfluorure.

Le catalyseur peut en outre comprendre des co-catalyseurs choisi parmi le Co, Zn, Mn, Mg, V, Mo, Te, Nb, Sb, Ta, P, Ni, Zr, Ti, Sn, Cu, Pd, Cd, Bi ou leurs mélanges. Lorsque le catalyseur est à base de chrome, le Ni, Mg et Zn sont avantageusement choisis comme co-catalyseur.

Le ratio atomique co-catalyseur/catalyseur est de préférence compris entre 0,01 et 5 .

Les catalyseurs à base de chrome sont particulièrement préférés.

Avantageusement les catalyseurs sont soumis à un traitement d'activation en présence d'un courant d'agent oxydant tel que l'air, l'oxygène ou le chlore.

Avantageusement les catalyseurs sont également soumis à une étape d'activation à l'aide d'un flux comprenant de l'acide fluorhydrique.

Selon un mode de réalisation, l'activation des catalyseurs peut être mise en oeuvre en deux étapes avec un traitement avec l'agent oxydant suivi de celui avec de l'HF.

Selon un autre mode de réalisation, l'activation des catalyseurs peut être mise en oeuvre en deux étapes avec un traitement avec de l'HF suivi de celui avec l'agent oxydant.

En fonction du catalyseur ou de la réaction, on peut effectuer plusieurs fois cette alternance (activations avec un traitement à l'air suivi de l'HF, de nouveau un traitement d'air suivi d'HF et ainsi de suite).

La température du traitement avec l'agent oxydant peut être comprise entre 250 et 500°C, de préférence entre 300 et 400°C pour une durée comprise entre 10 et 200 heures.

Celle du traitement avec l'HF peut être comprise entre 100 et 450°C, de préférence entre 200 et 300°C pour une durée comprise entre 1 et 50 heures.

Selon un autre mode de réalisation, l'activation des catalyseurs peut être mise en oeuvre en au moins une étape avec un traitement au mélange d'HF et d'agent oxydant. L'agent oxydant peut représenter entre 2 et 98 % molaire par rapport au mélange d'HF et agent oxydant et la température d'activation peut varier entre 200 et 450°C pour une durée comprise entre 10 et 200 heures.

L'activation de catalyseur peut être poursuivie par une réaction de fluoration en présence d'un agent oxydant, d'HF et d'au moins un composé choisi parmi un halopropane(s) de formules CX₃CHClCH₂X et CX₃CFXCH₃, et/ou au moins un halopropène(s) de formules CX₃CCl=CH₂, CClX₂CCl=CH₂ et CX₂=CClCH₂X, avec X représentant indépendamment un atome de fluor ou de chlore. Le ratio molaire HF/halopropane et/ou halopropène peut être compris entre 2 et 40. Le ratio molaire agent oxydant/ halopropane et/ou halopropène peut être compris entre 0,04 et 2,5 La durée de cette étape d'activation par fluoration peut être comprise entre 6 et 100 heures et la température comprise entre 300 et 400°C.

A l'issue de cette étape d'activation, le catalyseur est de préférence soumis à un traitement à l'air avant de mettre en oeuvre le procédé de fabrication du 2,3,3,3-tetrafluoropropène comprenant une étape (FCO) selon la présente invention.

Les étapes d'activation peuvent être mises en oeuvre à pression atmosphérique ou sous pression jusqu'à 20 bar.

Selon un mode de réalisation préféré de l'invention, on utilise un catalyseur mixte contenant à la fois du chrome et au moins un métal choisi parmi nickel, le zinc et le magnésium. Le ratio atomique (Ni/Zn/Mg)/Cr est généralement compris entre 0,01 et 5.

Avantageusement, le catalyseur est un catalyseur mixte de chrome et de nickel éventuellement supporté.

Le métal peut être présent sous forme métallique ou sous forme de dérivés, notamment oxyde, halogénure ou oxyhalogénure, ces dérivés, notamment halogénure et oxyhalogénure, étant obtenus par activation du métal catalytique. Bien que l'activation du métal ne soit pas nécessaire, elle est préférée.

Le support est à base d'aluminium. On peut citer plusieurs supports possibles comme l'alumine, l'alumine activée ou les dérivés d'aluminium. Ces dérivés d'aluminium sont notamment des halogénures ou oxyhalogénures d'aluminium obtenus par le procédé d'activation décrit ci-dessous.

Le catalyseur peut comprendre le chrome et du nickel, magnésium et/ou zinc sous une forme non-activée ou sous forme activée, sur un support qui a subi aussi l'activation du métal ou non.

Le support peut être préparé à partir d'alumine à porosité élevée. Dans une première étape l'alumine est transformée en fluorure d'aluminium ou en mélange de fluorure d'aluminium et d'alumine, par fluoration à l'aide d'air et d'acide fluorhydrique, le taux de transformation de l'alumine en fluorure d'aluminium dépendant essentiellement de la température à laquelle est effectuée la fluoration de l'alumine (en général entre 200°C et 450°C, de préférence entre 250°C et 400°C). Le support est ensuite imprégné à l'aide de solutions aqueuses de sels de chrome et de nickel, magnésium et/ou zinc ou à l'aide de solutions aqueuses d'acide chromique, de sel de nickel, magnésium et/ou zinc et de méthanol (servant de réducteur au chrome). Comme sels de chrome et de nickel, magnésium et/ou zinc, on peut employer des chlorures, ou d'autres sels tels que, par exemple, les oxalates, formiates, acétates, nitrates et sulfates ou le bichromate de nickel, magnésium et/ou zinc, pour autant que ces sels soient solubles dans la quantité d'eau susceptible d'être absorbée par le support.

Le catalyseur peut aussi être préparé par imprégnation directe de l'alumine (qui en général est activée) à l'aide des solutions des composés de chrome et de nickel, magnésium et/ou zinc, ci-dessus mentionnées. Dans ce cas, la transformation d'au moins une partie (par exemple 70% ou plus) de l'alumine en fluorure d'aluminium ou oxyfluorure d'aluminium s'effectue lors de l'étape d'activation du métal du catalyseur.

Les alumines activées susceptibles d'être utilisées pour la préparation du catalyseur sont des produits bien connus, disponibles dans le commerce. Elles sont généralement préparées par calcination d'hydrates d'alumine (hydroxydes d'aluminium) à une température comprise entre 300°C et 800°C. Les alumines (activées ou non) peuvent contenir des teneurs importantes (jusqu'à 1000 ppm) de sodium sans que cela nuise aux performances catalytiques.

De préférence, le catalyseur est conditionnée ou activé, c'est-à-dire transformé en constituants actifs et stables (aux conditions réactionnelles) par une opération préalable dite d'activation. Ce traitement peut être réalisé soit "in situ" (dans le réacteur de fluoration) ou bien dans un appareillage adéquat conçu pour résister aux conditions d'activation.

Après imprégnation du support, le catalyseur est séché à une température comprise entre 100°C et 350 °C, de préférence 220°C à 280°C en présence d'air ou d'azote.

Le catalyseur séché est ensuite activé en une ou deux étapes avec un agent oxydant et/ou l'acide fluorhydrique. Puis, est activé par une réaction de fluoration en présence d'un agent oxydant, d'HF et d'au moins un composé choisi parmi un halopropane(s) de formules CX₃CHClCH₂X et CX₃CFXCH₃ et/ou au moins un halopropène(s) de formules CX₃CCl=CH₂, CClX₂CCl=CH₂ et CX₂=CClCH₂X, avec X représentant indépendamment un atome de fluor ou de chlore. Le ratio molaire HF/composés organiques peut être compris entre 2 et 40. Le ratio molaire agent oxydant/ composés organiques peut être compris entre 0,04 et 2,5. La durée de cette étape d'activation par fluoration peut être comprise entre 6 et 100 heures et la température comprise entre 300 et 400°C.

La présente invention fournit également un procédé de fabrication du 2,3,3,3-tetrafluoropropène à partir du 2,3-dichloro-1,1,1-trifluoropropane (HCFC-243db), le 1,1,1,2,3-pentachloropropane (HCC-240db), le 2-chloro-3,3,3-trifluoro-1-propène (HCFO-1233xf) et/ou le 1,1,2,3-tetrachloropropène (HCO-1230xa) comprenant au moins une étape (FCO) tel que décrit ci-avant.

La présente invention fournit en outre un procédé de fabrication du 2,3,3,3-tetrafluoropropène en faisant réagir du 1,1,1,2,3-pentachloropropane (HCC-240db) avec de l'HF en phase gazeuse en présence d'un catalyseur pour donner un flux comprenant du 2-chloro-3,3,3-trifluoro-1-propène (HCFO-1233xf), du HFO-1234yf et éventuellement du 1,1,1,2,2-pentafluoropropane (HFC-245cb) ; le flux, après séparation du HFO-1234yf, est soumis à une étape (FCO).

L'étape de fluoration du HCC-240db et celle du flux, après séparation du HFO-1234yf, peuvent être mises en oeuvre soit dans un même réacteur soit dans deux réacteurs séparés.

L'oxygène présent dans l'étape (FCO) peut provenir de l'air ou de l'air enrichi en oxygène.

Le procédé selon la présente invention peut être mis en oeuvre en continu ou discontinu.

### PARTIE EXPERIMENTALE

### Catalyseur

Le catalyseur utilisé est à base de nickel/chrome avec un ratio atomique Ni/Cr = 1 supporté sur du fluorure d'aluminium et obtenu par imprégnation des solutions de sels de nickel et de chrome.

L'activation comprend les étapes suivantes:
Une étape de séchage est effectuée à pression atmosphérique sous courant d'azote introduit à environ 20 litre/h et à une température d'environ 220°C pendant 24 heures ;
Une première étape d'activation est effectué à une température d'environ 350°C sous un mélange azote et acide fluorhydrique en réduisant progressivement l'azote, jusqu'à observer un palier sous HF pur pendant 3 heures, puis sous courant d'air seul et ce pendant 64 heures ;
Une seconde étape d'activation avec de l'air, l'acide fluorhydrique et du HCFO-1233xf : ratio molaire HF/HCFO-1233xf =20 ; ratio molaire oxygène/HCFO-1233xf = 0,08 et temps de contact de 25s pendant 30 heures à 350°C ; puis un traitement sous courant d'air introduit à 1,5 litre/h pendant 64 heures avant de réaliser diverses réactions de fluoration.

### Schéma chronologique de l'activation :

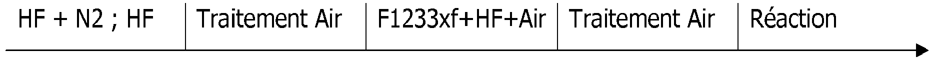

### Réaction

La réaction de fluoration est effectuée dans les conditions suivantes :
ratio molaire HF/ HCFO-1233xf = 24
ratio molaire oxygène/ HCFO-1233xf = 1,8 ou 0,6
temps de contact = 22 s
pression atmosphérique
température 350°C

Après 60 heures dans ces conditions, la conversion du HCFO-1233xf est de 50 % et la sélectivité en HFO-1234yf et HFC-245cb de 86 %.

Au bout de 1690 heures, la conversion est de 44 % et sélectivité en HFO-1234yf et HFC-245cb de 86 %.

On opère comme décrit précédemment à l'exception que la réaction de fluoration est mise en oeuvre avec un ratio HF/ HCFO-1233xf = 0,6.

Après 60 heures dans ces conditions, la conversion est du HCFO-1233xf est d'environ 32%.

Au bout de 250 heures de réaction, la conversion est de 28 % et une sélectivité en HFO-1234yf et HFC-245cb de 88%.

## Revendications

1. Procédé de fabrication du 2,3,3,3-tetrafluoropropène à partir des halopropanes de formules CX₃CHClCH₂X et CX₃CFXCH₃, halopropènes de formules CX₃CCl=CH₂, CClX₂CCl=CH₂ et CX₂=CClCH₂X, avec X représentant indépendamment un atome de fluor ou de chlore, **caractérisé en ce qu'**il comprend au moins une étape (ci-après nommée "étape FCO") au cours de laquelle du 2-chloro-3,3,3-trifluoro-1-propène éventuellement en mélange avec au moins un halopropane(s) de formule CX₃CHClCH₂X et CX₃CFXCH₃, et/ou au moins un halopropène(s) de formules, CClX₂CCl=CH₂ et CX₂=CClCH₂X, avec X représentant indépendamment un atome de fluor ou de chlore, réagit ou réagissent avec de l'HF en phase gazeuse en présence d'un catalyseur de fluoration à une température comprise entre 320 et 420°C, en présence d'un ratio molaire d'oxygène par rapport au 2-chloro-3,3,3-trifluoro-1-propène supérieur à 1 et inférieur ou égal à 2,5 et un ratio molaire HF par rapport à la totalité des composés à réagir compris entre 5 et 40.

2. Procédé selon la revendication 1 **caractérisé en ce que** le ratio molaire d'oxygène par rapport au 2-chloro-3,3,3-trifluoro-1-propène compris entre 1,25 et 2,5.

3. Procédé selon la revendication 1 ou 2 **caractérisé en ce que** le ratio molaire d'HF par rapport aux composés organiques à réagir est compris entre 10 et 40.

4. Procédé selon l'une quelconque des revendications 1 à 3 **caractérisé en ce que** la température de fluoration de l'étape FCO est comprise entre 340 et 400°C.

5. Procédé selon l'une quelconque des revendications 1 à 4 **caractérisé en ce que** l'étape FCO est mise en oeuvre à une pression comprise entre 0,5 et 20 bar, de préférence entre 1 et 7 bar.

6. Procédé selon l'une quelconque des revendications 1 à 5 **caractérisé en ce que** le 2-chloro-3,3,3-trifluoro-1-propène représente au moins 20% en poids du ou des composés organiques présents dans l'étape FCO.

7. Procédé selon l'une quelconque des revendications 1 à 6 **caractérisé en ce que** le catalyseur est massique ou supporté.

8. Procédé selon l'une quelconque des revendications 1 à 7 **caractérisé en ce que** le catalyseur est à base de chrome.

9. Procédé selon l'une quelconque des revendications 1 à 8 **caractérisé en ce que** le catalyseur comprend un co-catalyseur.

10. Procédé selon la revendication 9 **caractérisé en ce que** le co-catalyseur est choisi parmi le nickel, magnésium et zinc.

11. Procédé selon l'une quelconque des revendications précédentes **caractérisé en ce que** le catalyseur est soumis à au moins une étape d'activation à l'aide d'un flux comprenant de l'oxygène et/ou de l'HF.

12. Procédé selon la revendication 11 **caractérisé en ce que** l'activation est poursuivie par une réaction de fluoration en présence d'un agent d'oxydant, d'HF et d'au moins un composé choisi parmi les halopropanes de formules CX₃CHClCH₂X et CX₃CFXCH₃, halopropènes de formules CX₃CCl=CH₂, CClX₂CCl=CH₂ et CX₂=CClCH₂X, avec X représentant indépendamment un atome de fluor ou de chlore.

13. Procédé selon la revendication 12 **caractérisé en ce que** la température est comprise entre 300 et 400°C et la durée entre 6 et 100 heures.

14. Procédé selon l'une quelconque des revendications précédentes **caractérisé en ce que** les halopropanes ou halopropènes sont choisis parmi le 2,3-dichloro-1,1,1-trifluoropropane, le 1,1,1,2,3-pentachloropropane, le 2-chloro-3,3,3-trifluoro-1-propène et/ou le 1,1,2,3-tetrachloropropène.

15. Procédé selon l'une quelconque des revendications précédentes **caractérisé en ce qu'**il comprend au moins une étape réactionnelle au cours de laquelle le 1,1,1,2,3-pentachloropropane réagit avec de l'HF en phase gazeuse en présence d'un catalyseur pour donner un flux comprenant du 2-chloro-3,3,3-trifluoro-1-propène, du 2,3,3,3-tetrafluoropropène et éventuellement du 1,1,1,2,2-pentafluoropropane; au moins une étape pour séparer du 2,3,3,3-tetrafluoropropène du flux suivie d'une étape FCO du flux.

## Patentansprüche

1. Verfahren zur Herstellung von 2,3,3,3-Tetrafluorpropen ausgehend von Halogenpropanen der Formeln CX₃CHClCH₂X und CX₃CFXCH₃ und Halogenpropenen der Formeln CX₃CCl=CH₂, CCX₂CCl=CH₂ und CX₂=CClCH₂X, wobei X jeweils unabhangig ein Fluor- oder Chloratom darstellt, **dadurch gekennzeichnet, dass** es wenigstens einen Schritt (im Weiteren "FCO-Schritt" genannt) aufweist, in dessen Verlauf 2-Chlor-3,3,3-trifluor-1-propen, eventuell im Gemisch mit wenigstens einem Halogenpropan der Formeln CX₃CHClCH₂X und CX₃CFXCH₃, und/oder wenigstens einem Halogenpropen der Formeln CClX₂CCl=CH₂ und CX₂=CClCH₂X, wobei X jeweils unabhängig ein Fluor- oder Chloratom darstellt, mit HF in gasförmiger Phase in Gegenwart eines Fluorierungskatalysators bei einer Temperatur zwischen 320 und 420 °C reagiert oder reagieren, bei Vorliegen eines Molverhältnisses von Sauerstoff zu 2-Chlor-3,3,3-trifluor-1-propen, das größer als 1 und kleiner oder gleich 2,5 ist, und eines Molverhältnisses von HF zu der Gesamtheit der umzusetzenden Verbindungen, das zwischen 5 und 40 liegt.

2. Verfahren nach Anspruch 1, **dadurch gekennzeichnet, dass** das Molverhältnis von Sauerstoff zu 2-Chlor-3,3,3-trifluor-1-propen zwischen 1,25 und 2,5 liegt.

3. Verfahren nach Anspruch 1 oder 2, **dadurch gekennzeichnet, dass** das Molverhältnis von HF zu den umzusetzenden organischen Verbindungen zwischen 10 und 40 liegt.

4. Verfahren nach einem der Ansprüche 1 bis 3, **dadurch gekennzeichnet, dass** die Fluorierungsternperatur des FCO-Schrittes zwischen 340 und 400 °C liegt.

5. Verfahren nach einem der Ansprüche 1 bis 4, **dadurch gekennzeichnet, dass** der FCO-Schritt bei einem Druck zwischen 0,5 und 20 bar, vorzugsweise zwischen 1 und 7 bar, durchgeführt wird.

6. Verfahren nach einem der Ansprüche 1 bis 5, **dadurch gekennzeichnet, dass** 2-Chlor-3,3,3-trifluor-1-propen wenigstens 20 Gew.-% der organischen Verbindung oder Verbindungen darstellt, die in dem FCO-Schritt vorhanden sind.

7. Verfahren nach einem der Ansprüche 1 bis 6, **dadurch gekennzeichnet, dass** der Katalysator ein Vollkatalysator oder Trägerkatalysator ist.

8. Verfahren nach einem der Ansprüche 1 bis 7, **dadurch gekennzeichnet, dass** der Katalysator ein Katalysator auf Chrombasis ist.

9. Verfahren nach einem der Ansprüche 1 bis 8, **dadurch gekennzeichnet, dass** der Katalysator einen Cokatalysator umfasst.

10. Verfahren nach Anspruch 9, **dadurch gekennzeichnet, dass** der Cokatalysator aus Nickel, Magnesium und Zink ausgewählt ist.

11. Verfahren nach einem der vorhergehenden Ansprüche, dadurch gekenntzeichnet, dass der Katalysator wenigstens einem Aktivierungsschritt mithilfe eines Sauerstoff und/oder HF umfassenden Stroms unterzogen wird.

12. Verfahren nach Anspruch 11, **dadurch gekennzeichnet, dass** die Aktivierung mit einer Fluorierungsreaktion in Gegenwart eines Oxidationsmittels, von HF und wenigstens einer Verbindung, die aus den Halogenpropanen der Formeln CX₃CHClCH₂X und CX₃CFXCH₃ und Halogenpropenen der Formeln CX₃CCl=CH₂, CClX₂CCl=CH₂ und CX₂=CClCH₂X ausgewählt ist, wobei X jeweils unabhängig ein Fluor- oder Chloratom darstellt, fortgesetzt wird.

13. Verfahren nach Anspruch 12, **dadurch gekennzeichnet, dass** die Temperatur zwischen 300 und 400 °C und die Dauer zwischen 6 und 100 Stunden beträgt.

14. Verfahren nach einem der vorhergehenden Ansprüche, **dadurch gekennzeichnet, dass** die Halogenpropane oder Halogenpropene aus 2,3-Diehlor-1,1,1-trifluorpropan, 1,1,1,2,3-Pentachlorpropan, 2-Chlor-3,3,3-trifluor-1-propen und/oder 1,1,2,3-Tetrachlorpropen ausgewählt sind.

15. Verfahren nach einem der vorhergehenden Ansprüche, **dadurch gekennzeichnet, dass** es wenigstens einen Reaktionsschritt umfasst, in dessen Verlauf 1,1,1,2,3-Pentachlorpropan mit HF in gasförmiger Phase in Gegenwart eines Katalysators reagiert, um einen Strom zu erzeugen, der 2-Chlor-3,3,3-trifluor-1-propen, 2,3,3,3-fetrafluarpropen und eventuell 1,1,1,2,2-Pentafluorpropan umfasst; und wenigstens einen Schritt zum Trennen von 2,3,3,3-Tetrafluorpropen von dem Strom, gefolgt von einem FCO-Schritt des Stroms.

## Claims

1. Process for manufacturing 2,3,3,3-tetrafluoro-propene from halopropanes of formulae CX₃CHClCH₂X and CX₃CFXCH₃ and halopropenes of formulae CX₃CCl=CH₂, CClX₂CCl=CH₂ and CX₂=CClCH₂X, where X is independently a fluorine or chlorine atom, **characterized in that** it comprises at least one step (referred to as "FCO step" below) during which 2-chloro-3,3,3-trifluoro-1-propene, optionally mixed with at least one halopropane of formulae CX₃CHClCH₂X and CX₃CFXCH₃ and/or at least one halopropene of formulae CClX₂CCl=CH₂ and CX₂-CClCH₂X, where X is independently a fluorine or chlorine atom, react(s) with HF in the gas phase in the presence of a fluorination catalyst at a temperature of between 320 and 420°C in the presence of a molar ratio of oxygen to 2-chloro-3,3,3-trifluoro-1-propene of greater than 1 and less than or equal to 2.5 and a molar ratio of HF to all the compounds to be reacted of between 5 and 40.

2. Process according to Claim 1, **characterized in that** the molar ratio of oxygen to 2-chloro-3,3,3-trifluoro-1-propene is between 1.25 and 2.5.

3. Process according to Claim 1 or 2, **characterized in that** the molar ratio of HF to the organic compounds to be reacted is between 10 and 40.

4. Process according to any one of Claims 1 to 3, **characterized in that** the fluoridation temperature of the FCO step is between 340 and 400°C.

5. Process according to any one of Claims 1 to 4, **characterized in that** the FCO step is carried out at a pressure of between 0.5 and 20 bar, preferably between 1 and 7 bar.

6. Process according to any one of Claims 1 to 5, **characterized in that** the 2-chloro-3,3,3-trifluoro-1-propene represents at least 20% by weight of the organic compound(s) present in the FCO step.

7. Process according to any one of Claims 1 to 6, **characterized in that** the catalyst is a bulk or supported catalyst.

8. Process according to any one of Claims 1 to 7, **characterized in that** the catalyst is chromium-based.

9. Process according to any one of Claims 1 to 8, **characterized in that** the catalyst comprises a cocatalyst.

10. Process according to Claim 9, **characterized in that** the co-catalyst is chosen from nickel, magnesium and zinc.

11. Process according to any one of the preceding claims, **characterized in that** the catalyst is subjected to at least one activation step using a stream comprising oxygen and/or HF.

12. Process according to Claim 11, **characterized in that** the activation is continued by means of a fluorination reaction in the presence of an oxidizing agent, of HF and of at least one compound chosen from the halopropanes of formulae CX₃CHClCH₂X and CX₃CFXCH₃ and halopropenes of formulae CX₃CCl=CH₂, CClX₂CCl=CH₂ and CX₂=CClCH₂X, where X is independently a fluorine or chlorine atom.

13. Process according to Claim 12, **characterized in that** the temperature is between 300 and 400°C and the duration between 6 and 100 hours.

14. Process according to any one of the preceding claims, **characterized in that** the halopropanes or halopropenes are chosen from 2,3-dichloro-1,1,1-trifluoropropane, 1,1,1,2,3-pentachloropropane, 2-chloro-3,3,3-trifluoro-1-propene and/or 1,1,2,3-tetrachloropropene.

15. Process according to any one of the preceding claims, **characterized in that** it comprises at least one reaction step during which 1,1,1,2,3-pentachloropropane reacts with HF in the gas phase in the presence of a catalyst so as to give a stream comprising 2-chloro-3,3,3-trifluoro-1-propene, 2,3,3,3-tetrafluoropropene and, optionally, 1,1,1,2,2-pentafluoropropane; and at least one step for separating 2,3,3,3-tetrafluoropropene from the stream, followed by an FCO step on the stream.
